# EUROPEAN PATENT SPECIFICATION

(11) **EP 3 125 946 B1**
(45) Date of publication and mention of the grant of the patent: **07.12.2022**
(21) Application number: 15770224.2
(22) Date of filing: 29.03.2015
(51) Int. Cl.: A61K 48/00, A61K 31/56, A61K 47/50, C07C 22/02, C07C 13/66

(54) **COMPOUNDS AND METHODS FOR TRANS-MEMBRANE DELIVERY OF MOLECULES**
VERBINDUNGEN UND VERFAHREN ZUR TRANSMEMBRANABGABE VON MOLEKÜLEN
COMPOSÉS ET PROCÉDÉS POUR L'ADMINISTRATION TRANSMEMBRANAIRE DE MOLÉCULES

(30) Priority: 28.03.2014 US 201461971548 P; 13.04.2014 US 201461978903 P; 25.05.2014 US 201462002870 P; 06.06.2014 US 201462008509 P; 14.12.2014 US 201462091551 P
(43) Date of publication of application: 08.02.2017
(73) Proprietor: Aposense Ltd., 49170 Petach-tikva (IL)
(72) Inventor: ZIV, Ilan, Kfar Saba 4441805 (IL)
(74) Representative: Pearl Cohen Zedek Latzer Baratz UK LLP
(86) International application number: PCT/IL2015/000019
(87) International publication number: WO 2015/145417

(56) References cited:
- WO-A1-97/40679
- WO-A1-98/50041
- WO-A1-2014/127052
- WO-A2-2005/077968
- FR-A1- 2 846 969
- US-A- 6 028 066
- US-A1- 2011 123 457
- MARIA CRISTINA BELLUCCI ET AL: "Multicomponent Synthesis of Peptide-Sugar Conjugates Incorporating Hexafluorovaline", ADVANCED SYNTHESIS & CATALYSIS, vol. 352, no. 16, 30 September 2010 (2010-09-30), pages 2791-2798, XP055408800, DE ISSN: 1615-4150, DOI: 10.1002/adsc.201000489
- VACLAV JANOUT ET AL: "Molecular Umbrella Conjugate for the Ocular Delivery of siRNA", BIOCONJUGATE CHEMISTRY, vol. 25, no. 2, 19 February 2014 (2014-02-19), pages 197-201, XP055351708, ISSN: 1043-1802, DOI: 10.1021/bc400506m
- SANTIAGO GRIJALVO ET AL: "Synthesis of Oligonucleotides Carrying Amino Lipid Groups at the 3'-End for RNA Interference Studies", THE JOURNAL OF ORGANIC CHEMISTRY, vol. 75, no. 20, 15 October 2010 (2010-10-15), pages 6806-6813, XP055408915, ISSN: 0022-3263, DOI: 10.1021/jo101143j
- YUE XUYI ET AL: "Synthesis and characterization of fluorinated conjugates of albumin", JOURNAL OF FLUORINE CHEMISTRY, ELSEVIER, NL, vol. 152, 4 February 2013 (2013-02-04), pages 173-181, XP028576415, ISSN: 0022-1139, DOI: 10.1016/J.JFLUCHEM.2013.01.026
- ZHONG-XING JIANG ET AL: "The Design and Synthesis of Highly Branched and Spherically Symmetric Fluorinated Macrocyclic Chelators", SYNTHESIS, vol. 2008, no. 2, 1 January 2008 (2008-01-01), pages 215-220, XP055408344, STUTTGART, DE. ISSN: 0039-7881, DOI: 10.1055/s-2007-1000857
- J BLAZEJEWSKI ET AL: "Synthesis, Characterization and Biological Evaluation of 7[alpha]-Perfluoroalkylestradiol Derivatives", BIOORGANIC & MEDICINAL CHEMISTRY, vol. 11, no. 3, 6 February 2003 (2003-02-06), pages 335-345, XP055408464, GB ISSN: 0968-0896, DOI: 10.1016/S0968-0896(02)00457-1
- SCHILLER R ET AL: "DSC measurements on full thickness mice skin: An additional tool to investigate permeation enhancement of highly lipophilic drugs", JOURNAL OF THERMAL ANALYSIS AND CALORIMETRY, KLUWER ACADEMIC PUBLISHERS, DORDRECHT, NL, vol. 77, no. 2, 1 August 2004 (2004-08-01) , pages 497-510, XP019255124, ISSN: 1572-8943
- SUN S1, ADEJARE A.: 'Fluorinated molecules as drugs and imaging agents in the CNS.' CURR TOP MED CHEM. vol. 6, no. 14, 01 January 2006, pages 1457 - 64, XP055358842
- ZHONG-XING JIANG ET AL: "The Synthesis of a Geminally Perfluoro- tert -butylated beta-Amino Acid and its Protected Forms as a Potential Pharmacokinetic Modulator and Reporter for Peptide-Based Pharmaceuticals", JOURNAL OF ORGANIC CHEMISTRY, vol. 72, no. 4, 1 February 2007 (2007-02-01), pages 1464-1467, XP055659733, US ISSN: 0022-3263, DOI: 10.1021/jo0616308
- BENJAMIN C. BUER ET AL: "Perfluoro- tert -butyl-homoserine as a sensitive 19 F NMR reporter for peptide-membrane interactions in solution : A SENSITIVE 19 F NMR REPORTER FOR PEPTIDE-MEMBRANE INTERACTIONS", JOURNAL OF PEPTIDE SCIENCE., vol. 19, no. 5, 1 May 2013 (2013-05-01), pages 308-314, XP055659213, GB ISSN: 1075-2617, DOI: 10.1002/psc.2501
- HUNT J A ET AL: "2-Arylbenzoxazoles as CETP inhibitors: Substitution and modification of the +/--alkoxyamide moiety", BIOORGANIC & MEDICINAL CHEMISTRY LETTERS, PERGAMON, AMSTERDAM, NL, vol. 20, no. 3, 1 February 2010 (2010-02-01), pages 1019-1022, XP026861858, ISSN: 0960-894X [retrieved on 2010-01-19]

## Description

### FIELD OF THE INVENTION

The invention relates to a delivery system for delivery of molecules across biological membranes into cells, optionally with subsequent intracellular entrapment.

### BACKGROUND

Protein pathology is a common denominator in the etiology or pathogenesis of many medical disorders, ranging from malfunction of a mutated protein, to pathological gain of function, wherein a specific protein acquires a novel property which renders it toxic. Conceptually, inhibition of the synthesis of these of proteins by gene therapy may hold promise for patients having such protein anomaly.

One of the major advances of recent years is the concept of silencing a specific gene by RNA interference, using small interfering RNA (siRNA). RNA interference is based on short (≈19-27 base pairs) double-stranded RNA sequences capable of acting, in concert with cellular biological systems (among others, the Dicer protein complex which cleaves double-stranded RNA to produce siRNA, and the RNA-induced silencing complex (RISC)), to inhibit translation and mark for degradation specific mRNA sequences, thus inhibiting gene expression at the translational stage. The use of antisense oligonucleotide (ASO), a short sequence (usually 13-25 nucleotides) of unmodified or chemically modified DNA molecules, complementary to a specific mRNA, has also been suggested to inhibit the expression and block the production of a respective specific target protein.

However, albeit the tremendous potential benefits of such approaches for medical care, trans-membrane delivery of such macromolecules remains a substantial challenge, due to the relatively large and highly-charged structures of siRNA (average MW of 13kD, about 40 negatively-charged phosphate groups). Therefore, trans-membrane delivery of siRNA requires overcoming a very large energetic barrier.

The membrane dipole potential is the electric potential that exists within any phospholipid membrane, between the water/membrane interface and the membrane center (positive inside). It is assumed to be generated due to the highly ordered carbonyl groups of the phospholipid glyceryl esteric bonds. Its amplitude is about 220-280mV. Since it is in a highly hydrophobic environment, of dielectric constant of 2-4, it translates into a very strong electric field of 10⁸-10⁹ V/m. Conceivably, the membrane dipole potential and related intra-membrane electric field are highly important for the function of membrane proteins. The dipole potential is likely to have an important physiological role in determining the conformation and activity of membrane proteins. However, to date, the dipole potential has not been recruited for medical uses.

Various methods have been developed for delivery of oligonucleotides across biological membranes. These methods include viral vectors as well as non-viral delivery systems, such as cationic lipids or liposomes. However, to date, use of these methods has been largely limited to applications *in vitro,* or for focal administration *in vivo,* for example, by direct injection into the eye or direct administration to the lung. For example, electroporation is an effective and widely-used method for delivery of macromolecules across biological barriers. According to this method, an external electric field is applied to a cell suspension, leading to collision of charged target molecules with the membrane, subsequent temporary and focal membrane destabilization, and consequent passage of the macromolecules into the cell. However, to date, electroporation is used mainly *in vitro.* Electroporation *in vivo* has met limited success, and was attempted only to specific organs (e.g., muscle, lung), where external electrodes could be inserted into the target organ.

In conclusion, delivery of macromolecules, such as oligonucleotides and other therapeutic agents, through cell membranes and other biological barriers such as the Blood-Brain-Barrier, still presents a substantial unmet need, and systemic delivery (namely delivery via intravenous administration or *per os*) of such macromolecules, still remains a huge, unaddressed challenge.

Bellucci et al. describe a multicomponent synthesis of peptide-sugar conjugates incorporating hexafluorovaline (Advanced Synthesis and Catalysis, vol. 352, no. 16, 2010, p. 2791-2798, XP055408800, DOI: 10.1002/adsc.201000489).

Janout et al. describe a molecular umbrella conjugate for the ocular delivery of siRNA (Bioconjugate Chemistry, vol. 25, no. 2, 2014, p. 197-201, XP055351708, DOI: 10.1021/bc400506m).

FR2846969 A1 (Salles et al.) describes new modified active agent derivatives with improved bioavailability comprising parent drug plus fluorinated hydrocarbon chain and recognition or hydrophilizing moiety.

Grijalvo et al. describe the synthesis of oligonucleotides carrying amino lipid groups at the 3'-end for RNA interference studies (The Journal of Organic Chemistry, vol. 75, no. 20, 2010, p. 6806-6813, XP055408915, DOI: 10.1021/jo101143j).

Xuyi et al. describe the synthesis and characterization of fluorinated conjugates of albumin (Journal of Fluorine Chemistry, vol. 152, 2013, p. 173-181, XP028576415, DOI: 10.1016/J.JFLUCHEM.2013.01.026).

WO 98/50041 A1 (IMARX PHARMACEUTICAL CORP) describes novel lipid soluble steroid prodrugs, compositions comprising steroid prodrugs, and uses of the same.

US Patent No. 6,028,066 describes novel prodrugs comprising fluorinated amphiphiles, compositions comprising the novel prodrugs, and methods of use of the prodrugs and compositions.

Jiang et al. describe the design and synthesis of highly branched and spherically symmetric fluorinated macrocyclic chelators (Synthesis, vol. 2008, no. 2, 2008, p. 215-220, XP055408344, DOI: 10.1055/s-2007-1000857).

Blazejewski et al. describe the synthesis, characterization and biological evaluation of 7-[alpha]-perfluoroalkylestradiol derivatives (Bioorganic and Medicinal Chemistry, vol. 11, no. 3, 2003, pages 335-345, XP055408464, DOI: 10.1016/S0968-0896(02)00457-1).

Schiller et al. describe DSC measurements on full thickness mice skin and further detail an additional tool to investigate permeation enhancement of highly lipophilic drugs (Journal of Thermal Analysis and Calorimetry, vol. 77, no. 2, 2004, p. 497-510, XP019255124).

WO2005/077968 A2 (Innoventus Project AB) describes novel compounds which are 7-alpha-substituted 17-alkylene-16 alpha-hydroxy steroidal estrogens and also relates to the use of the compounds as a medicament, and for the treatment of estrogen dependent disorders.

Jiang et al. describe the synthesis of a geminally perfluoro-tert-butylated [beta]-amino acid and its protected forms as a potential pharmacokinetic modulator and reporter for peptide-based pharmaceuticals (Journal of Organic Chemistry, vol 72, no. 1, 2007, p. 1464-1467, XP055659733, DOI: 10.1021/jo0616308).

Buer et al. describe perfluoro-tert-butyl-homoserine as a sensitive 19 F NMR reporter for peptide-membrane interactions in solution (Journal of Peptide Science, vol. 19, no. 5, 2013, p. 308-314, XP055659213, DOI 10.1002/psc.2501).

Hunt et al. describe 2-arylbenzoxazoles as CETP inhibitors and disclose the substitution and modification of the +/--alkoxyamide moiety (Bioorganic and Medicinal Chemistry Letters, vol. 20, no. 3, 2010, p. 1019-1022, XP026861858).

### SUMMARY

The present invention provides a compound for trans-membrane delivery of a drug, the compound having a structure as set forth in Formula (VI): 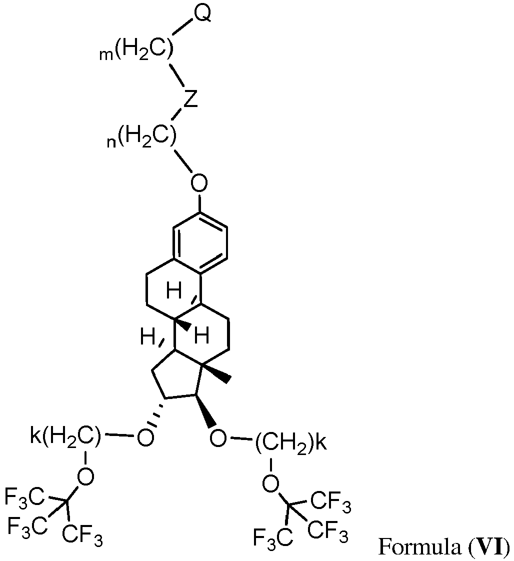 wherein
**n** and **m** are integers, each individually selected from null and 1, 2, 3, 4, 5, 6, 7, 8, 9, 10, 11, 12, 13, 14, 15, 16, 17, 18, 19 or 20;
**k** is an integer, selected from 2, 3, 4, 5, 6 or 7;
**Z** is null or -S-S-; and
**Q** is a linkage point to a drug;
or having a structure as set forth in Formula (**VII**): 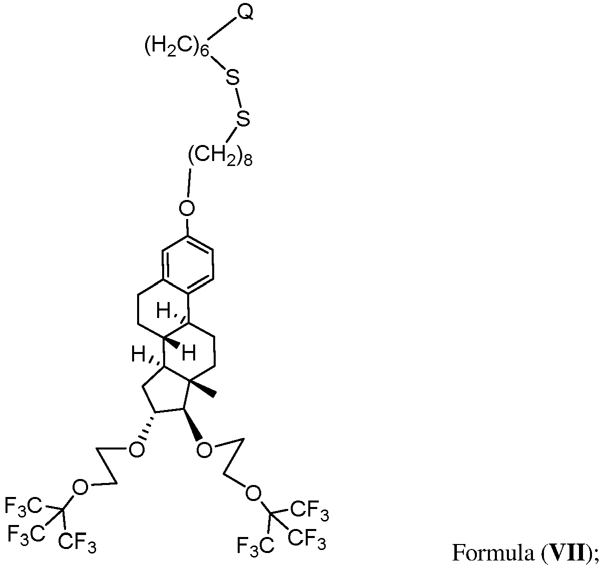
wherein **Q** is a linkage point to a drug;
or having a structure as set forth in Formula (**VIIa**): 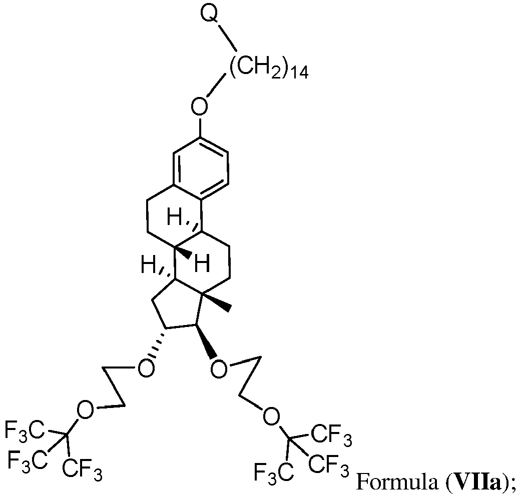
wherein **Q** is a linkage point to a drug;
or having a structure as set forth in Formula **(VIII):** 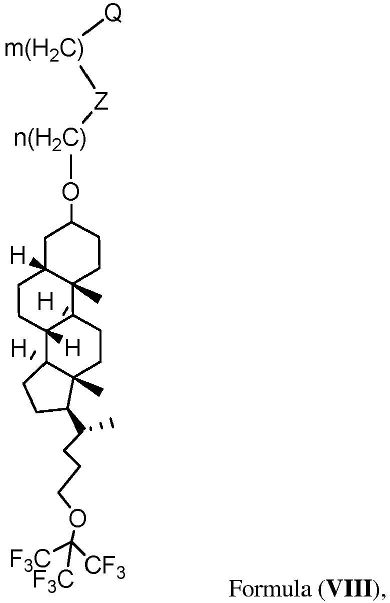 wherein
**n** and **m** are integers, each individually selected from null and 1, 2, 3, 4, 5, 6, 7, 8, 9, 10, 11, 12, 13, 14, 15, 16, 17, 18, 19 or 20;
**Z** is selected from null or -S-S-; and
**Q** is a linkage point to a drug;
or having a structure as set forth in Formula **(IX):** 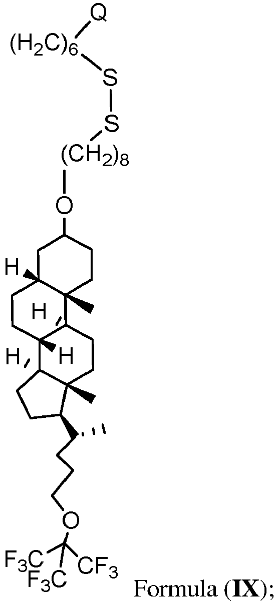
wherein **Q** is a linkage point to a drug;
or having a structure as set forth in Formula **(IXa):** 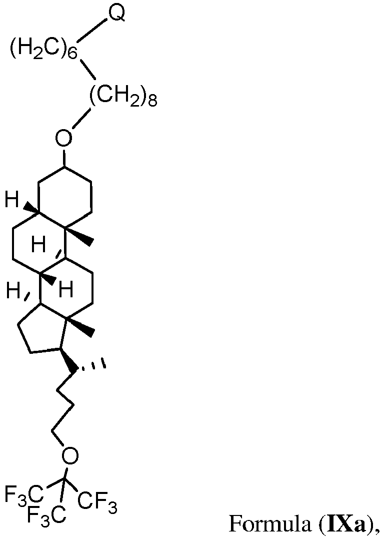
wherein **Q** is a linkage point to a drug;
or a pharmaceutically acceptable salt, hydrate, solvate or metal chelate thereof.

The present invention provides a pharmaceutical composition comprising a compound of the invention, and a pharmaceutically-acceptable salt or carrier.

The present invention provides a compound of the invention for use in delivering a drug across a biological membrane.

In an embodiment, the drug is a small-molecule drug or a macromolecule drug, selected from a natural or modified RNA or DNA, a small interfering RNA (siRNA), an antisense oligonucleotide (ASO) or a therapeutic protein.

In an embodiment, the therapeutic protein comprises a CRISPR protein, such as the Cas9 protein.

In an embodiment, the drug comprises a Dicer substrate, comprising double-stranded RNA of 25-30 nucleotides, that includes a sequence for silencing a target gene.

In an embodiment, delivering the drug is for treating a medical disorder, wherein the drug is useful for the treatment of the medical disorder.

In an embodiment, the medical disorder is cancer, a toxic insult, an ischemic disease, an infectious disease, a protein storage disease, trauma, an immune-mediated disease, a degenerative disease, or a neurodegenerative disorder such as Alzheimer's disease, motor neuron disease, Parkinson's disease, Huntington's disease, multiple sclerosis or Creutzfeldt-Jacob disease.

### BRIEF DESCRIPTION OF THE FIGURES

The invention will now be described in connection with certain examples and embodiments, in a non-limiting manner, with reference to the following illustrative figures, so that it can be more fully understood. In the drawings:
**Fig. 1A** is a schematic presentation of the principle of asymmetrical polarity, underlying the activity of compounds according to embodiments of the invention;
**Fig. 1B** schematically depicts structural motifs of the molecules of the invention as demonstrated by a compound according to Formula IX;
**Fig. 2** schematically illustrates a potential mechanism of action of a conjugate according to embodiments of the invention;
**Fig. 3** schematically illustrates a mechanism for entrapment of siRNA within the cytoplasm, utilizing the Dicer enzyme;
**Fig. 4** shows an exemplary structure of a conjugate, comprising a "molecular motor" of the invention, conjugated to the Cas9 protein; and
**Figs. 5A** and **5B** show the results of the biological performance of compounds according to embodiments of the invention; **Fig 5A** shows fluorescent microscopy results and **Fig. 5B** shows results of quantitative analysis by ELISA reader at 24 hours of incubation.

### DETAILED DESCRIPTION OF THE INVENTION

The present invention relates to novel compounds that may act as a delivery system for drugs across biological membranes, such as phospholipid cell membranes, into the cytoplasm. Compounds according to embodiments of the invention comprise novel, rationally-designed "molecular motors" designed to move within phospholipid membranes, from the membrane / water interface to the membrane core, utilizing the internal membrane electric field which is related to the membrane dipole potential. When attached to a drug, said delivery system acts to pull the drug towards the membrane core, and to facilitate its trans-membrane movement. Among others, this delivery system is designed for the delivery of therapeutic macromolecules: proteins or oligonucleotides, the latter being single or double-strands of DNA or RNA. Among others, said delivery system is designed for the delivery of antisense oligonucleotides (ASO), siRNA and therapeutic proteins such as the Cas9 Protein.

Accordingly, the present invention provides a compound for trans-membrane delivery of a drug, the compound having a structure as set forth in Formula **(VI):** 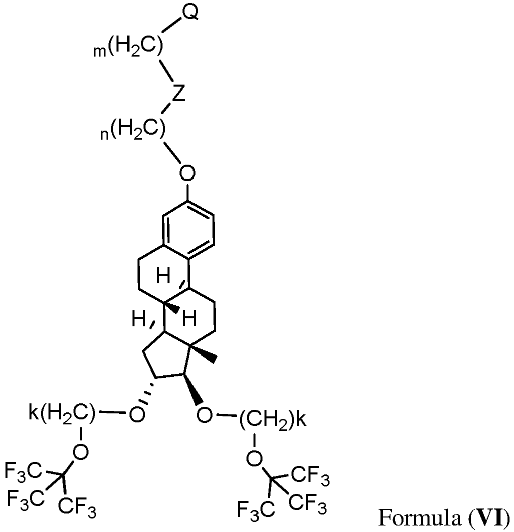 wherein
**n** and **m** are integers, each individually selected from null and 1, 2, 3, 4, 5, 6, 7, 8, 9, 10, 11, 12, 13, 14, 15, 16, 17, 18, 19 or 20;
**k** is an integer, selected from 2, 3, 4, 5, 6 or 7;
**Z** is null or -S-S-; and
**Q** is a linkage point to a drug.

The invention also provides a compound for trans-membrane delivery of a drug having a structure as set forth in Formula (**VII**): 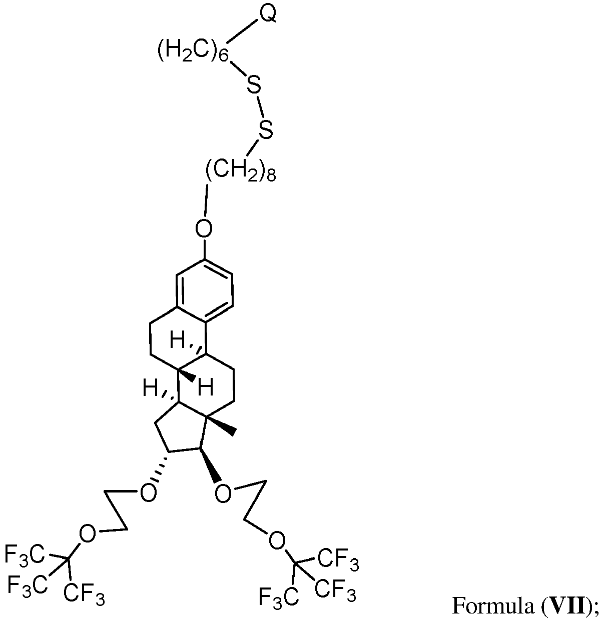 wherein Q is a linkage point to a drug.

The invention also provides a compound for trans-membrane delivery of a drug having a structure as set forth in Formula (**VIIa**): 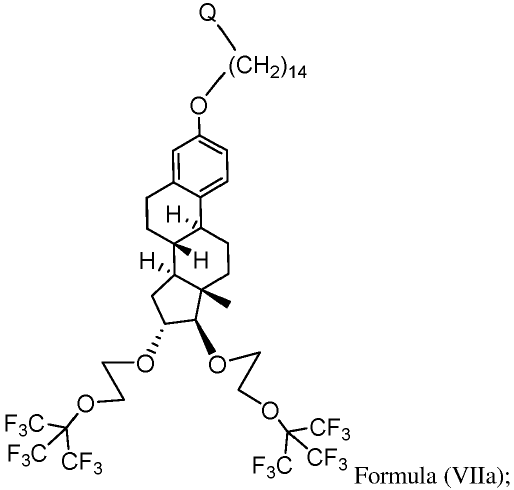 wherein **Q** is a linkage point to a drug.

The invention also provides a compound for trans-membrane delivery of a drug having a structure as set forth in Formula **(VIII):** 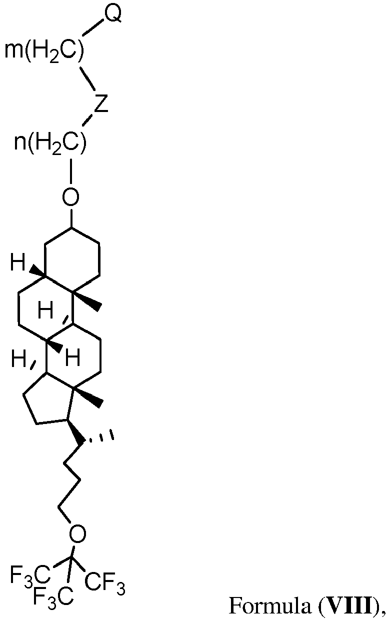 wherein
**n** and **m** are integers, each individually selected from null and 1, 2, 3, 4, 5, 6, 7, 8, 9, 10, 11, 12, 13, 14, 15, 16, 17, 18, 19 or 20;
**Z** is selected from null or -S-S-; and
**Q** is a linkage point to a drug.

The invention also provides a compound for trans-membrane delivery of a drug having a structure as set forth in Formula **(IX):** 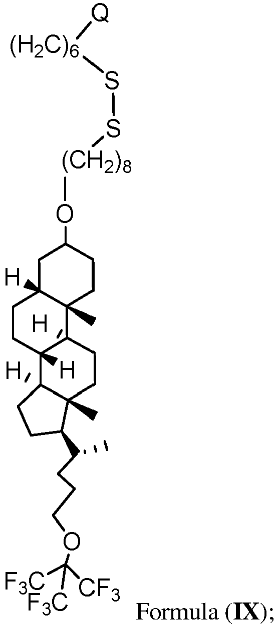 wherein **Q** is a linkage point to a drug.

The invention also provides a compound for trans-membrane delivery of a drug having a structure as set forth in Formula **(IXa):** 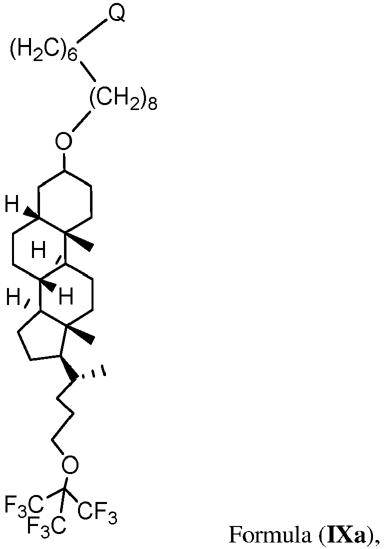 wherein **Q** is a linkage point to a drug.

The invention also includes a pharmaceutically acceptable salt, hydrate, solvate or metal chelate of the above compounds.

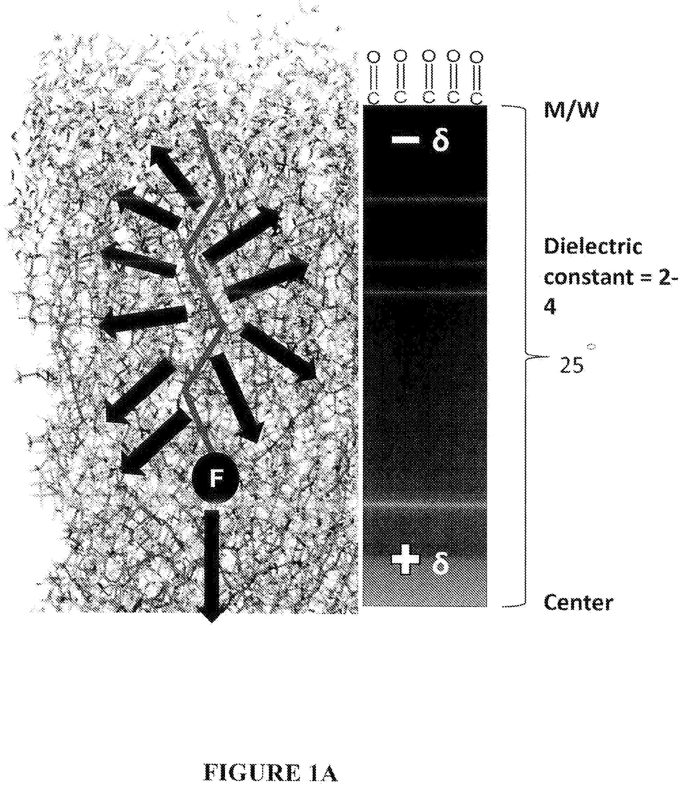

One of the principles underlying the structures of compounds according to embodiments of the invention is the principle of "asymmetrical polarity", concerning hydrophobic, uncharged molecules that according to their *logP,* partition into biological membranes (see **Fig. 1A****).** The molecules are polar, with their partial charges distributed unequally: the partial negative charge is highly localized and focused, while the partial positive charge is dispersed along hydrocarbon chains within the molecule. In addition, the partial positive charge is also masked, through London type interactions with adjacent hydrocarbon chains within the membrane lipid milieu (London dispersion forces). Consequently, as schematically illustrated in **Fig.1A****,** a molecule according to embodiments of the invention moves in the membrane milieu as a negatively-charged molecule. Since the membrane internal electric field has the negative pole at the membrane/water interface, and the positive pole at the membrane center, the molecule moves in the related electric field towards the membrane center, and when attached to a cargo (e.g., siRNA or ASO, a therapeutic protein or other medicament), it pulls the cargo to the membrane center.

In addition, when an optional cleavable group (*e.g.,* a disulfide group or an oligonucleotide sequence cleavable by a Dicer enzyme) is included in a molecule according to embodiments of the invention, it may act to trap the cargo (*e.g.,* siRNA or ASO or other medicament) in the cytoplasm of the target cell, and also assist in maintaining a concentration gradient of the conjugate across the cell membrane. The term "cleavable group" in the context of the present invention therefore relates to a chemical moiety, capable of undergoing spontaneous or an enzyme-mediated cleavage in certain physiological conditions, such as changes in pH or changes in red-ox state. Examples for cleavable groups are ester, thio-ester, amide, carbamate, disulfide [-(S-S)-] ether (-O-) or thioether (-S-). Conceptually, a cleavable group may assist in entrapment of the drug within its target cell following its trans-membrane passage, or assist in maintaining a concentration gradient across the biological membranes.

For example, in the case of a conjugate according to an embodiment of the invention, that comprises an siRNA, ASO or a therapeutic protein, and a disulfide group; once inside the cytoplasm, the prevailing ambient reductive environment will act to reduce the disulfide bond to -SH groups, with release of the cargo from the delivery moiety. Devoid of the "molecular motors" (delivery moiety), the cargo macromolecule will then be captured in the cytoplasm, where for example, in the case of siRNA, will be ready for interaction with the RNA-induced silencing complex (RISC) in order to silence the expression of a specific gene. According to embodiments of the invention, said gene may encode for a protein taking part in the etiology or pathogenesis of a specific disease.

According to one embodiment of the invention, the cargo is a therapeutic protein to be delivered across cell membranes cells in order to exert beneficial therapeutic effects on intracellular protein targets.

The field of Protein Drugs for Intracellular Targets (PDIT) is a novel field derived in part from the completion of the Human Genome Sequencing Project, which allows identification of a huge number of intracellular targets for potential medical interventions through administration of protein drugs, gene silencing, RNA or DNA editing, or protein replacement therapy. Conceptually, such therapeutic strategy can be useful for almost any medical disorder. Specific, highly attractive candidate proteins within the PDIT field are the CRISPR (*clustered regularly interspaced short palindromic repeats*)-*related* proteins, and specifically, the Cas9 Protein. This very recently-discovered protein, is initially a bacterial protein, naturally-used by the bacteria as an anti-viral mechanism. Practically, this protein can be loaded by any RNA sequence, entailing specificity in directing the protein specifically to any locus within the genome. Potentially, such locus may be a site harboring a mutated, defective gene. At that site, the Cas9 protein will then induce an accurate cut, *i.e.,* will induce a double-strand break of the DNA. Naturally-occurring DNA repair mechanisms are then activated to repair that site of the mal-functioning gene. Therefore, this protein enables highly effective gene editing (adding, disrupting or changing the sequence of specific genes) and gene regulation and repair, applicable to species throughout the tree of life. By delivering the Cas9 protein and an appropriate guide RNA into a cell, the organism's genome can be cut at any desired location, and subjected to editing and repair.

As exemplified below, an embodiment of the invention includes conjugates comprising one or more "molecular motors" and the Cas9 Protein or related proteins that have a role in editing DNA or RNA. Another embodiment of the invention includes a therapeutic protein, administered as a replacement therapy for a disease that is associated with relatively reduced levels of a specific, physiologically-important protein.

Compounds according to embodiments of the invention typically include a hydrophobic (octanol to water partition co-efficient (logP>1)), dipolar, uncharged chemical moiety, designed according to the principle of asymmetrical polarity (explained above). As discussed, the unique structure of a molecule, being hydrophobic, neutral, but comprising focused partial negative charges and dispersed partial positive charges, creates a vectorial system when put in the force field of the membrane, by which the molecule moves within the phospholipid membrane, from the membrane / water interface to the membrane core. When attached to a drug this molecule will respectively pull the drug to the membrane core.

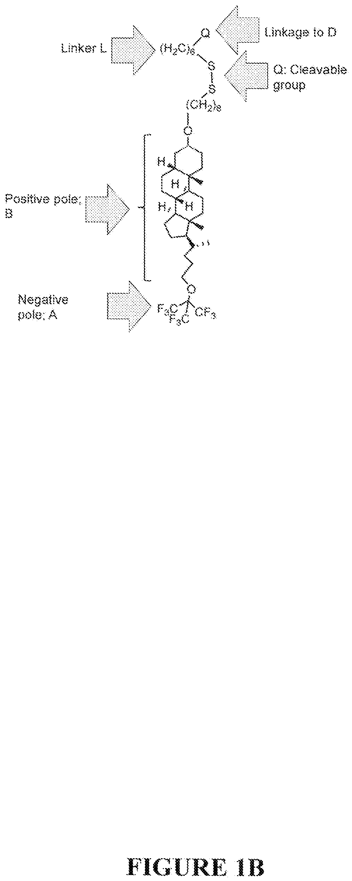

As schematically illustrated in **Fig. 1B** compounds according to embodiments of the invention (demonstrated, for example, by a compound according to **Formula IX)** typically include a "molecular motor" which typically is combination of the following structural elements:

**(i). A negative pole (A):** typically comprising at least 1 electronegative atom, selected from a halogen (e.g., fluorine atom(s)) and oxygen; wherein in the case that the pole comprises several electronegative atoms, they are arranged in space in a focused, spherical (or near spherical) arrangement. Due to the electron-withdrawing properties of said atoms, and their arrangement in space, the negative pole of the compound is an electron-rich focus.

**(ii). A positive pole (B):** comprising relatively electropositive atoms, selected from carbon, silicon, boron, phosphor and sulfur; arranged to enable maximal interaction with adjacent hydrocarbon chains when put in a phospholipid membrane, preferably through arrangement as an aliphatic or aromatic structure of linear, branched or cyclic chains, or combinations thereof. In one embodiment of the invention, the positive pole comprises a linear saturated hydrocarbon chain, or a steroid moiety, such as cholesterol, bile acids, estradiol, estriol or combinations thereof.

In addition to the "molecular motor" a compound of the invention may include one or more linkers **(L)** and cleavable groups **(Q).** A compound may be conjugated with or linked to a drug through a linker or cleavable group.

The compounds of the invention may be conjugated to drugs, such as proteins or oligonucleotides (e.g., siRNA or ASO) for use in the treatment of medical disorders, such as degenerative disorders, cancer, toxic or ischemic insults, infections or immune-mediated disorders, in which specific proteins may play a role in disease etiology or pathogenesis, and wherein silencing the expression of the respective gene(s) through siRNA or antisense mechanisms may have beneficial effects in inhibiting disease-related processes.

For example, the conjugates of the invention may be used as antisense therapy, which is a form of medical treatment comprising the administration of a strand or a double stand of nucleic acid (DNA, RNA or a chemical analogue) that will bind to DNA encoding for a specific protein, or to the respective messenger RNA (mRNA). This treatment acts to inhibit the expression of the gene and prevent the production of the respective protein. Alternatively, the conjugates may comprise therapeutic proteins such as the Cas9 protein.

The terms "drug" or "medicament" in the context of the present invention relate to a chemical substance, that when administered to a patient suffering from a disease, is capable of exerting beneficial effects on the patient. Said beneficial effects can be amelioration of symptoms, or counteracting the effect of an agent or a substance that play(s) a role in the disease process. The drug may comprise a small molecule or a macromolecule, such as a protein or strands of RNA or DNA, administered to inhibit gene expression. Among others, the drug may include siRNA or ASO. In some embodiments, the drug is aimed to treat degenerative disorders, cancer, ischemic, infectious or toxic insults, or immune-mediated disorders.

Compounds of the invention may be conjugated to a drug. Pharmaceutical compositions comprising said conjugates may be used for the treatment of medical disorders.

According to some embodiments, the compounds and pharmaceutical compositions of the invention may be used to achieve more effective performance of replacement protein therapy or gene therapy, such as siRNA or antisense therapy (ASO) *in vivo.*

A conjugate may be advantageous in improving delivery of siRNA, ASO or therapeutic proteins through cell membranes or through the Blood-Brain-Barrier, thus improving the performance of siRNA or ASO in one or more aspects, such as, for example, efficacy, toxicity, or pharmacokinetics.

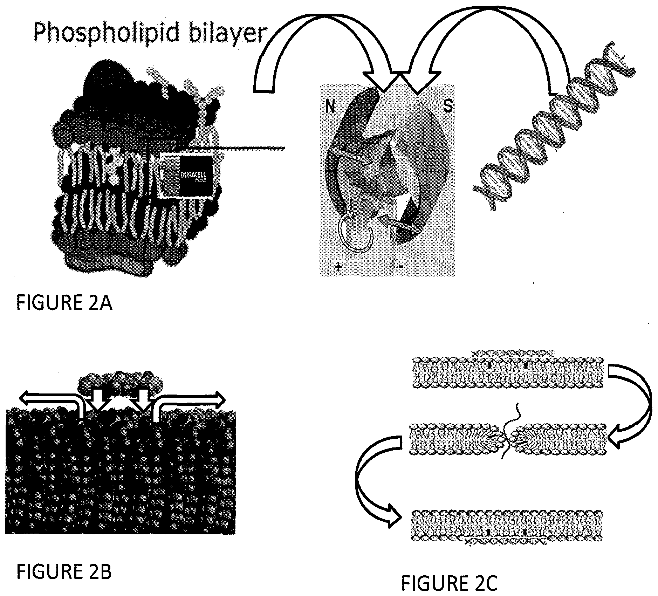

As described above, a potential mechanism of action (MOA), conjugates comprising a drug such as an siRNA or a therapeutic protein, when positioned within a phospholipid membrane, the "molecular motor" (e.g., as described above) acts to pull the drug towards the membrane core. Consequently, focal destabilization of the membrane may ensue, with lateral movement of phospholipid head-groups and generation of transient membrane pores, through which trans-membrane passage of the drug can take place. This potential MOA is schematically summarized in **Fig. 2****.**

In an example, schematically presented in **Fig. 2****,** a conjugate comprises a cargo, being siRNA, ASO or a therapeutic protein, and disulfide groups, for entrapment of the cargo in the cytoplasm. In a first stage (A) the "motor" is moved from the membrane surface to the membrane core, due to the principle of asymmetrical polarity, energized by the internal membrane electric field.

In a second stage (B) the macromolecule, linked to the "motor" is forced to approach the membrane surface, thus perturbing hydration shells. Consequently, there is lateral movement of the phospholipid head-groups, and formation of transient membrane pores, through which the macromolecule is delivered into the cell. Subsequent closure of the transient pore is thermodynamically-favored (C).

In another embodiment, entrapment of siRNA in the cytoplasm may include: **(*i*).** administration of a Dicer substrate, comprising double-stranded RNA of 25-30 nucleotides, that includes a sequence for silencing a target gene, wherein the "molecular motor(s)" are attached to the 3'-end of the sense (*passenger*) strand and / or to the 5'-end of the antisense (*guide*) strand; ***(ii).*** administration of a conjugate according to embodiments of the invention which will cause trans-membrane delivery of a drug (e.g., siRNA) and cleavage of the dsRNA by the Dicer enzyme, thus removing the "molecular motor(s)" from the conjugate, and liberating the siRNA. The siRNA, due to its numerous negative charges, will be entrapped in the cytoplasm and will be able to interact with the RISC complex for silencing the target gene.

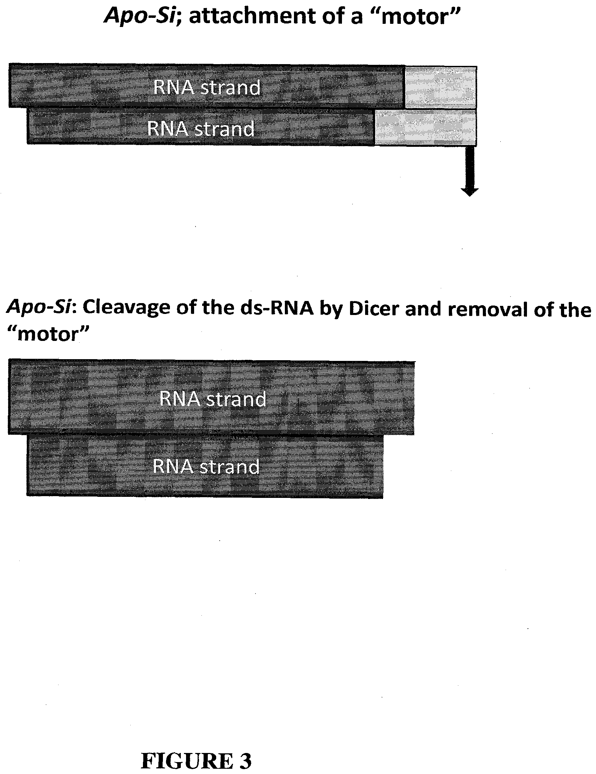

In one embodiment schematically illustrated in **Fig. 3** a mechanism for entrapment of siRNA within the cytoplasm, utilizing the Dicer enzyme is shown. A Dicer substrate, comprising double-stranded RNA of 25-30 nucleotides that includes a sequence for silencing of a target gene is shown. A "molecular motor" (arrow) is attached to the 3'-end of the sense (*passenger*) strand, and / or to the 5'-end of the antisense (*guide*) strand of the siRNA designed to silence a target gene. Following the trans-membrane delivery, cleavage of the dsRNA by the Dicer enzyme takes place, removing the "molecular motors" away from the conjugate, and liberating the siRNA to interact with the RISC complex, for silencing of the target gene. By this mechanism, the siRNA, due to its numerous negative charges, is thus entrapped in the cytoplasm.

The linkage of drugs to moieties of the molecule can be through ether, ester, amide, thioester, thioether or carbamate groups. In the case that a drug is an oligonucleotide, linkage can be to the nucleobase, to the ribose moiety (e.g., through the 2', 3' or 5' positions), or to the phosphate moiety of the nucleotide; linkage can be either to a terminal or to a non-terminal nucleotide of the oligonucleotide chain; in the case that the drug is a protein, its linkage to the other moieties of the molecule can be through linkage to side chain(s) of amino acids of the protein, such as lysine, glutamate or aspartate.

The term "oligonucleotide", in the context of the invention, may include DNA or RNA molecules, each being a single-stranded or double-stranded sequence of one or more nucleotides. Each nucleotide comprises a nitrogenous base (nucleobase), a five-carbon sugar (ribose or deoxyribose), and a phosphate group. The nucleobases are selected from purines (adenine, guanine) and pyrimidines (thymine, cytosine, uracil). In addition, the term may refer to modified forms of nucleotides, wherein the modification may be at the backbone of the molecule (e.g., phosphorothioate) or in the nucleobase (e.g., methylation at the 2' position of the ribose group in RNA). These modifications may enable properties such as improved stability or improved pharmacokinetics of the oligonucleotide, and the use of such modified oligonucleotides is also within the scope of the invention.

Linkage can be to the nucleobase, to the ribose moiety (e.g., through the 2', 3' or 5' positions), or linkage to the phosphate moiety of the nucleotide. The linkage can be either to a terminal or to a non-terminal nucleotide of the oligonucleotide chain.

In one embodiment, the present invention provides a compound for use in the specific inhibition of gene expression *in vivo* or the compound can be used in a method for the specific inhibition of gene expression *in vitro;* comprising the utilization of a conjugate, or a respective pharmaceutical composition, where a drug is siRNA or ASO, designed to silence the expression of a specific gene that encodes a pathogenic protein, that has a role in the etiology or pathogenesis of a disease.

Accordingly, the conjugates may be used for the treatment of a medical disorder. Embodiments also disclose a compound of the invention for use in medical treatment, comprising the administration to a patient in need adequate amounts of a pharmaceutical composition according to embodiments of the invention. In one embodiment, the administered pharmaceutical composition may include siRNA or an anti-sense oligonucleotide, active in inhibiting the expression of a gene which encodes for a specific pathogenic (*i.e.,* disease-related) protein.

Embodiments of the invention may further include pharmaceutical compositions, comprising a conjugate that includes a molecule according to any of **VI, VII, VIIa, VIII, IX** or **IXa,** and a pharmaceutically-acceptable salt or carrier.

In an embodiment, the compound of the present invention is for use in the specific inhibition of gene expression, *in vivo.* In one embodiment, the invention may include utilization of a compound according to **Formula VI, VII, VIIa, VIII, IX, IXa** or a respective pharmaceutical composition, wherein the compound is conjugated to a drug which is siRNA or an ASO, designed to silence the expression of a specific gene wherein said gene encodes for a pathogenic protein, having a role in the etiology or pathogenesis of a disease; or a therapeutic protein.

Conjugates may be used for the treatment of a medical disorder by administration to a patient in need thereof. Thus, in an embodiment the invention comprises a compound according to **Formula VI, VII, VIIa, VIII, IX, IXa** conjugated to a drug for use in the treatment of the respective medical disorder.

In one embodiment, the conjugated compound is for use in genetic treatment with siRNA or ASO by administration to a patient in need thereof. Thus, in an embodiment the invention comprises a compound of the invention according to **Formula VI, VII, VIIa, VIII, IX, IXa** conjugated to a drug, wherein the drug is siRNA, an ASO or a therapeutic protein, for use in inhibiting gene expression having a role in a disease of the specific patient.

In another embodiment of the invention, the drug is a protein to be delivered across biological phospholipid membranes into cells, or through biological barriers such as the blood-brain barrier.

In another embodiment of the invention, the drug is a normal protein, administered as a replacement therapy, *e.g.,* to replace a mutated malfunctioning protein.

In another embodiment, the drug is a protein that has as role in gene regulation, including, among others, proteins that have a role in DNA or RNA editing (adding, disrupting or changing the sequence of specific genes). In one embodiment, said protein may be a member of the CRISPRs (clustered regularly interspaced short palindromic repeats)-related proteins. Specifically, said protein can be or may comprise the Cas9 protein (CRISPR associated protein **9),** an RNA-guided DNA nuclease enzyme, or an analogue thereof.

In one embodiment, a compound of the present invention is for use in the genetic treatment of a medical disorder by administration to a patient in need thereof. Thus, in an embodiment the invention comprises a compound according to **Formula VI, VII, VIIa, VIII, IX, IXa** conjugated to a drug, where the drug is a CRISPR protein, such as Cas9, administered together with an appropriate guide oligonucleotide, thus achieving delivery of said protein, loaded by the respective guide oligonucleotide into the cells, where they can exert their genome editing activity. A guide oligonucleotide, in this context, is a sequence of RNA or DNA that guides the Cas-9 protein to a specific locus (place) on the DNA, in order to repair a defect in the genetic material. In the case of the **Cas9** protein, the guide oligonucleotide is RNA.

Therefore, conjugated compounds according to embodiments of the invention, and the respective pharmaceutical compositions, may be beneficial, among others, for use in treating medical disorders, selected, among others, from cancer, toxic insults, ischemic disease, infectious disease, protein storage disease, trauma, immune-mediated disease, or a degenerative disease.

In the field of neurological disorders, conjugates according to embodiments of the invention may be useful, among others, for use in the treatment of neurodegenerative disorders, such as Alzheimer's disease, Motor Neuron Disease, Parkinson's disease, Huntington's disease, multiple sclerosis and Creutzfeldt-Jacob disease.

### EXAMPLES

### Example 1: A general method for synthesis of conjugates comprising oligonucleotides, according to embodiments of the invention:

Initially, a gene to be silenced is chosen based on its role in disease etiology or pathogenesis. Then, based on bio-informatic methodologies known in the art, the nucleotide sequence (typically 19-21 base pairs for a RISC substrate, and 25-29 for a Dicer substrate) of a respective siRNA of the DNA sequence of ASO is determined.

Synthesis is carried out in the 3' to 5' direction. Solid phase synthesis is applied, using phosphoramidite building blocks, derived from protected 2'-deoxynucleosides (dA, dC, dG, and T), ribonucleosides (A, C, G, and U), or chemically modified nucleosides, e.g. LNA (locked nucleic acids) or BNA (bridged nucleic acids). The building blocks are sequentially coupled to the growing oligonucleotide chain, in the order required by the sequence of the desired siRNA or ASO.

Following the construction of the oligonucleotide, a compound according to the embodiments of the invention **(E,** as defined above) is added as one of the building blocks of the oligonucleotide. Optionally, the compound may be added in its precursor form as described above. For addition of the compound at the 5'-end of the oligonucleotide, the compound in its precursor form may include a phosphoramidite moiety. For addition of the compound at the 3'-end of the oligonucleotide, the compound in its precursor form may include acetylene or azide moieties. Usually, the process is fully automated. Upon completion of the assembly of the chain, the product is released from the solid phase to solution, deprotected, and collected. The desired conjugate is then collected and isolated by high-performance liquid chromatography (HPLC), to obtain the desired oligonucleotides in high purity. In the case of siRNA, each of a complementary RNA strand is synthesized separately, and then annealing of the two strands is performed, to yield the desired siRNA double-stranded RNA.

### Example 2: A method for synthesis of a specific exemplary compound according to an embodiment of the invention:

Synthesis of an exemplary compound according to embodiments of the invention is initiated from the bile acid lithocholic acid.

Perfluoro-tertbutanol is commercially-available, and may serve as an example of moiety A as described above.

The synthesis proceeds as described in **Scheme I** below. In this example, the **E** moiety is designed to be linked to the 5'-end of the oligonucleotide, and therefore a phosphoramidite moiety is added at the last step of the synthesis.

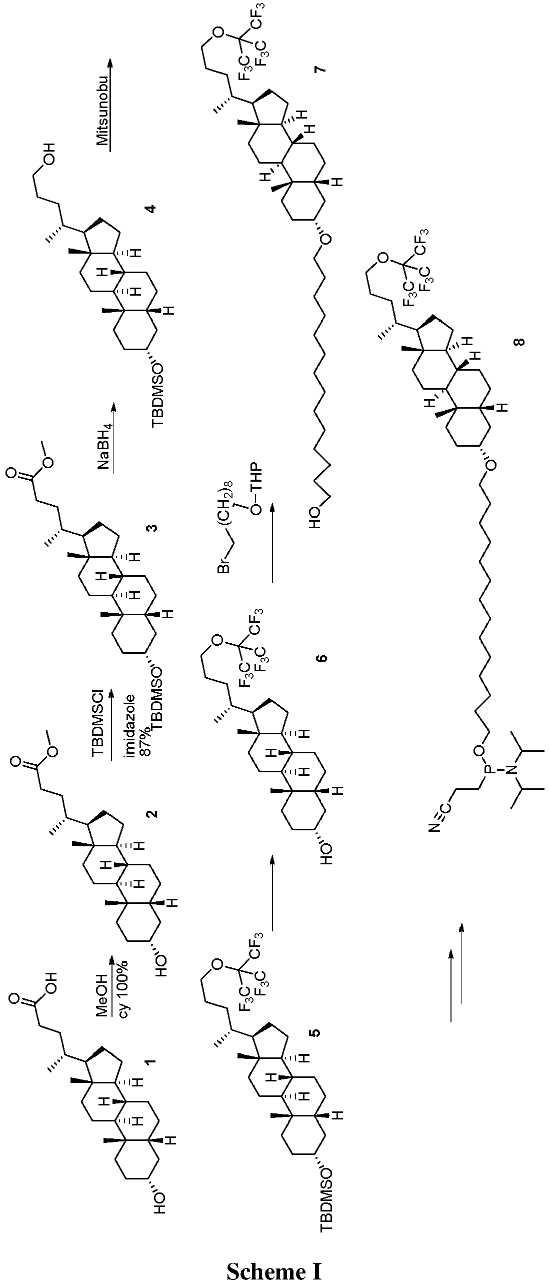

25 g of material **1** were converted to 26 g (quantitative) methyl ester (material **2).** 26 g of material **2** were reacted with TBDMSC1 NS 29 g (87%) NMR pure material **3** were obtained. Reduction of material **3** (29 g) to material **4** with NaBH₄ THF/MeOH gave, after work up and purification, 25 g of material **4** (85%) by NMR with still some traces of material **3.** Mitsunobu reaction of material **4** with perfluoro t-butanol gave, after work-up column chromatography and trituration from MeOH, 33.5 g (92%) of material **5,** which was deprotected thereafter, to give steroid **6.** Steroid **6** (2.5 g) was then coupled to THP-protected bromotetradecanol. The coupling took 3 days and 4 equivalents of THP-protected bromotetradecanol were needed to reach complete conversion. The product was purified by column chromatography. After removal of the protecting group (THP) with MeOH/1,4-dioxane (HC1, 4 N)/THF, product **7** was purified by column chromatography to remove impurities. The product **7** (1.5 g, c.y 48%) was obtained as white solid. Product **7** was then converted into the requested compound **8,** by attachment of the phosphoramidite group.

### Reference Example 3: Structures of precursors and respective compounds attached to oligonucleotides

### a. 5' modification:

### Precursor:

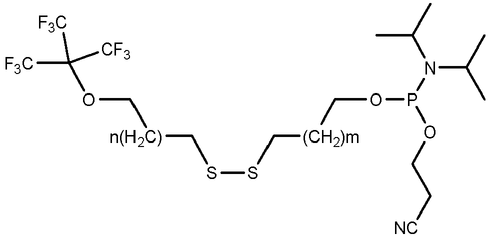

**As attached to an oligonucleotide:**

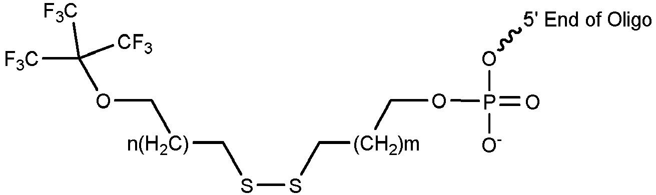

### b. 3' modification:

### Precursor:

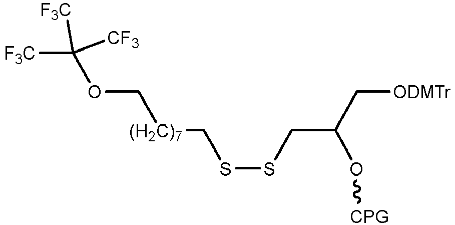

### DMT= Dimethoxytrityl;

CPG= Controlled Pore Glass (CPG) as a solid support for the synthesis of the oligonucleotide.

### As attached to an oligonucleotide:

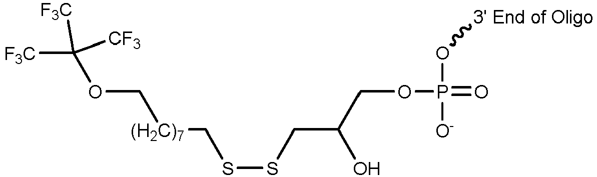

### c. 5' modification; attachment of compound to a nucleobase (e.g., thymine):

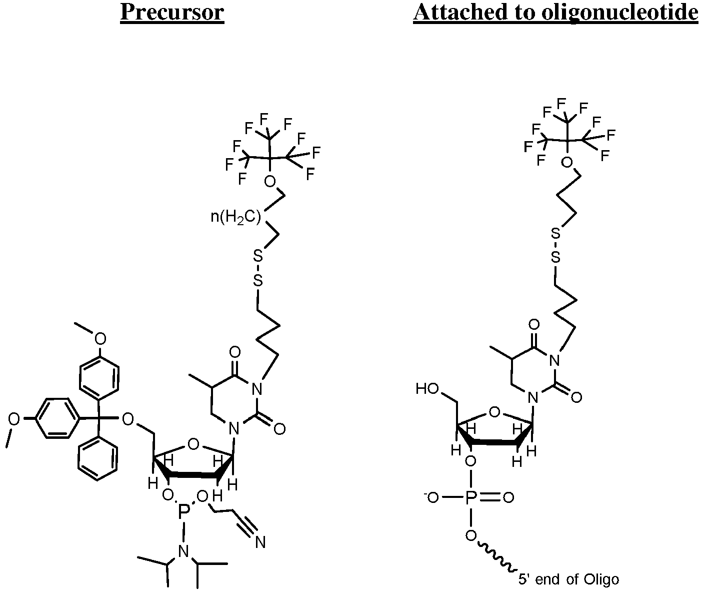

### Example 4: An exemplary structure of a compound conjugated with Cas9 protein according to embodiments of the invention

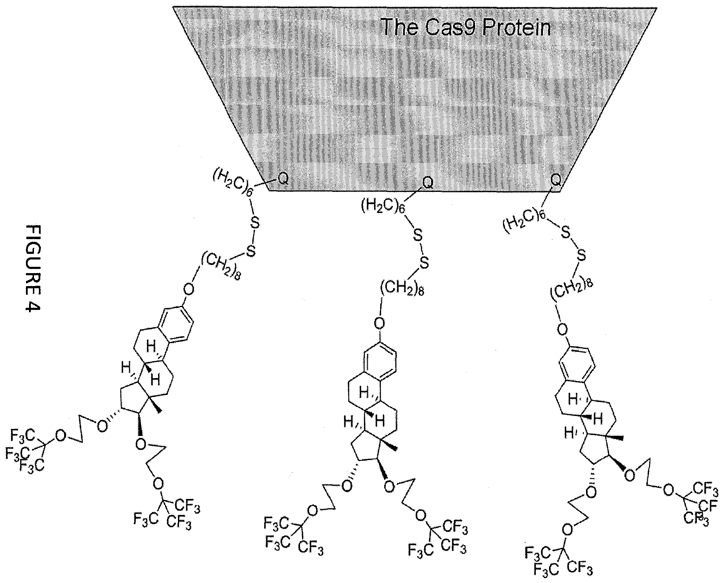

A structure of a "molecular motor" of the invention, conjugated to the Cas9 protein is schematically illustrated in **Fig. 4****.** The structure is based on crystallographic studies of the structure of the Cas9 protein *(*Nishimazu FG, et al., Cell 156:935-949, 2014*).* Compounds **E** according to embodiments of the invention will be attached through their **Q** group to the protein, binding to lysine side-chains on the protein surface. For attachment, active esters will be used. The alcohol will be converted to a carbonate active ester that preferentially reacts with nitrogen (protein lysine side-chains) over oxygen (water). Reaction will be performed according to the following **Scheme II:**

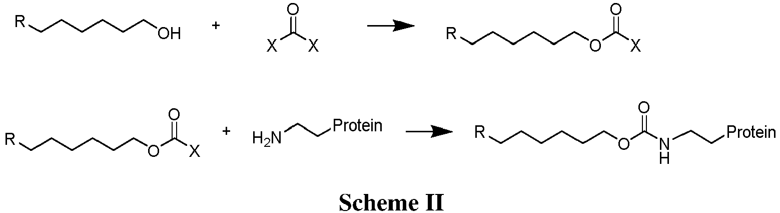

Possible derivatizing agents are:
a) Phosgene: linkage is through chloroformate ester.
b) Disuccinimidyl carbonate (X = N-hydroxysuccinimide): linkage is through a succinimidyl carbonate.
c) Carbonyldiimidazole (CDI, X = Imidazole): linkage is through imidazolyl carbamate.

Protein labeling with any of these groups takes place in an amine-free (not Tris), slightly basic buffer (pH = 8-9). The linkage point is hydrophobic, thus requiring a co-solvent (normally DMF or DMSO) for the reaction with proteins. High reactivity means shorter reaction times but also lower nitrogen over oxygen selectivity and shorter lifetime in aqueous buffer. With all active esters, the product is a carbamate which may be susceptible to enzymatic cleavage. Of these three options above, carbonyldiimidazole has the highest nitrogen over oxygen selectivity, as well as the simplest synthesis. On the other hand, protein derivatization may take longer (probably overnight). The number of E moieties per protein molecule is determined by pre-setting the desired molar ratios.

### Example 5: Cellular uptake and localization of Cv3-labled SS 29-mer DNA sequence in vitro according to embodiments of the invention .

### Cell culture:

NIH -3T3 cell lines stably transfected with GFP were obtained from Cell Biolabs, San-Diego, USA (cat-AKR214).

The cells were grown in Dulbecco modified Eagle's medium (DMEM; Gibco cat- 41965) supplemented with 10% fetal bovine serum (FBS; Biological Industries cat-04-011-1A), 2 mM L-glutamine (Biological Industries cat- 03-020-1B), 1% Pen-Strep (Biological Industries cat- 03-031-1B) and10 µg/ml Blasticidin (Enzo cat- ALX-380-089) at 37°C in a humidified incubator containing 5% CO₂.

### Assessment of 29-mer DNA delivery into the cells; In-vitro assay:

One day before the experiment NIH-3T3-GFP cells in an exponential growth phase were plated in 24-well plates at a density of 4.5×10⁴ cells/well with DMEM plus supplements growth medium (500 (µl/well) without antibiotics. Each of the Cy3-labeled oligonucleotide was diluted in 100 µl/well of Opti-Mem (Life technologies-Cat. 31985062) and added to the cells in final concentration varying from 40 to 100 nM.

The accumulation of the Cy3-labeled oligonucleotide delivery system within the cells was evaluated 2, 4, 8 and 24 h post incubation. Following the incubation period the cells were washed with Hank's Buffered Salt Solution (HBSS buffer; Biological Industries) and subjected to analysis.

Detection and quantification of Cy3-positive population was performed using Tecan Infinite^{®} 200 PRO multimode reader (excitation wavelength 548±4.5 nm and emission 580±10 nm).

The trans-membrane delivery of a compound according to embodiments of the invention attached to the Cy3-ssDNA oligonucleotide (29nt) (Syncom, the Netherlands; IDT, USA) was compared to the control Cy3-ssDNA oligonucleotide (IDT, USA) and expressed as percentage of Cy3- positive cells compared to untreated cells.

### Fluorescence microscopy:

NIH-3T3 GFP cells at a density of 4.5×10⁴/well were plated 24 h before the experiments. The growth medium was replaced by fresh medium containing a compound according to embodiments of the invention attached to the Cy3-ssDNA oligonucleotide (29nt) (40-100 nM dissolved in DNase/RNase free water) or Control Cy3-ssDNA (IDT, USA) and incubated 2, 4, 8 and 24 h at 37°C.

Following incubation, the cells were washed with HBSS and visualized using Olympus fluorescent microscope (BX51TF; Olympus Optical, U.K.), with UV illumination from a mercury lamp. The Cy3-fluorophore was visualized with excitation at 470-495 nm and emission at 590 nm. The GFP-fluorophore was visualized with excitation at 530-550 nm and emission at 510-550 nm.

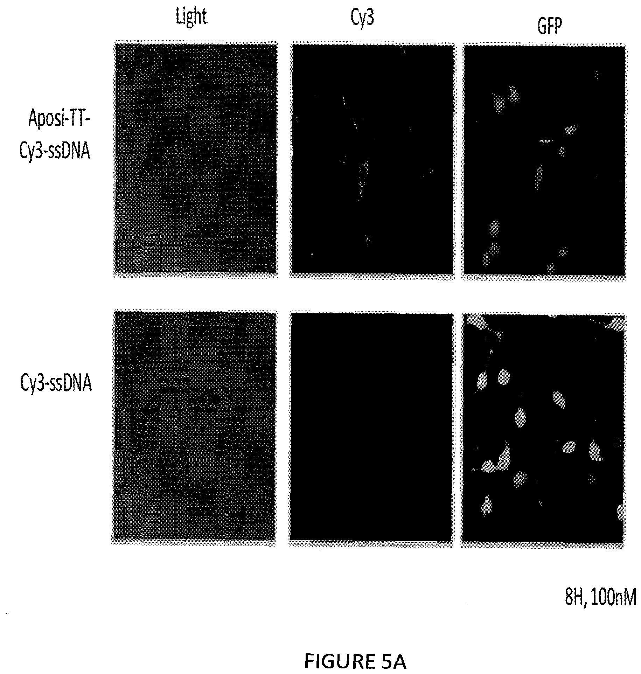

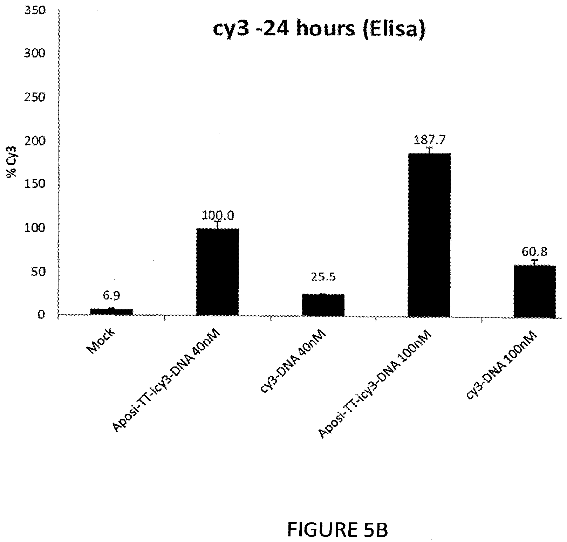

The biological performance of a conjugate according to embodiments of the invention, in delivery across phospholipid membranes, into cells, of an ASO of ss 29-mer DNA, linked at its 5'-end, through a phosphate bond to an E moiety (in this example a compound of **Formula VIIa**) according to embodiments of the invention, is described in **Figs. 5A** and **5B****.** The construct also had an iCy3-fluorophore at the 5'-end. Delivery was evaluated in NIH-3T3 cells, stably transfected with the GFP protein.

**Fig. 5A** shows the results of 3T3-GFP cells that were incubated with a compound according to embodiments of the invention attached to the Cy3-ssDNA ("E-conjugate") or Cy3-ssDNA at a concentration of 100nM for a period of 8 hours. The cells were thereafter analyzed by fluorescent microscopy (x20 magnitude).

The E-conjugate is shown on the upper panel, while the lower panel is of a control ASO, devoid of the compound according to embodiments of the invention ("E moiety").

As shown, while the control construct, devoid of the E moiety did not manifest any significant delivery into the cells, the E-conjugate manifested uptake into nearly all cells. Images were acquired at 8 hours of incubation at 37°C. Incubation with 100nM of the E-conjugate is shown.

### Quantitative analysis by ELISA

The 3T3-GFP cells were incubated with E-conjugate or Cy3-ssDNA at varying concentrations (40nM and 100nM) for 24 hours. Cells were washed and assessed by ELISA fluorimeter. Cy3 fluorescent levels were compared to 3T3-GFP untreated cells. As shown in **Fig. 5B****,** at doses of 40nM and 100nM, the conjugate manifested 4-fold and 3-fold increased delivery into the cells, as compared to controls.

### Example 6: Knock-down of mRNA by siRNA in vitro

Inhibitory activity of the 25 - 27 bp dsRNAs in cells stably expressing the GFP (green fluorescent protein) reporter gene is evaluated. For this purpose, stably transfected NIH 3T3 cells expressing GFP are transfected with 25 - 27-mer dsRNA duplexes (each at 10-40 nM). To obtain a quantitative estimate of the duration of GFP gene knock-down, a time-course experiment, observing GFP expression after transfection will be carried- out.

One day before the experiment NIH-3T3-GFP cells in an exponential growth phase will be plated in 24-well plates at a density of 4.5×10⁴ cells/well with DMEM plus supplements growth medium (500 µl/well) without antibiotics. Each of the Cy3-labeled oligonucleotide was diluted in 100 µl/well of Opti-Mem (Life technologies-Cat. 31985062) and added to the cells in final concentration varying from 40 to 100 nM.

The knock down of mRNA of GFP within the cells will be evaluated 24, 48 and 72 h post incubation. Following the incubation period the cells were washed with Hank's Buffered Salt Solution (HBSS buffer; Biological Industries) and subjected to analysis.

Detection and quantification of GFP-positive population was performed using Tecan Infinite^{®} 200 PRO multimode reader (excitation wavelength 488±4.5 nm and emission 535±10 nm).

The knock down activity of a compound according to embodiments of the invention attached to the 25 - 27 bp dsRNAs (Syncom, the Netherlands; IDT, USA) will be compared to the control 25 - 27 bp dsRNAs (IDT, USA) and expressed as percentage of GFP- positive cells compared to untreated cells.

### Example 7: A mechanism for intracellular entrapment of siRNA, comprising administration of a Dicer substrate

In an embodiment of the invention, an *in vitro* method for entrapment of siRNA in the cytoplasm is based on the activity of the enzyme Dicer, in processing double-stranded RNA, cutting it at the size of 19-21 base pairs, suitable for interaction with the RISC Complex for gene silencing. Said *in vitro* method comprises: *(i).* administration of a Dicer substrate, comprising double-stranded RNA of 25-30 nucleotides, that includes a sequence for silencing of a target gene, wherein compounds according to embodiments of the invention are attached to the 3'-end of the sense (*passenger*) strand, and / or to the 5'-end of the antisense (*guide*) strand; *(ii).* trans-membrane delivery of the siRNA; and *(iii).* cleavage of the dsRNA by the Dicer enzyme, thus liberating the siRNA to interact with the RISC complex, for silencing the target gene.

For Dicer cleavage assays *in vitro,* siRNA duplexes (100 pmol) may be incubated in 20 ml of 20 mM Tris pH 8.0, 200 mM NaCl, 2.5 mM MgCl2, with 1 unit of recombinant human Dicer (Stratagene) for 24 h. A 3-ml aliquot of each reaction (15 pmol RNA) will be separated in a 15% non-denaturing polyacrylamide gel, stained with GelStar (Ambrex) and visualized using UV excitation. Electrospray-ionization liquid chromatography mass spectroscopy (ESILCMS) of duplex RNAs before and after treatment with Dicer will be done using an Oligo HTCS system (Novatia), which consists of ThermoFinniganTSQ7000, Xcalibur data system, ProMass data processing software and Paradigm MS4 HPLC (Michrom BioResources).

## Claims

1. A compound for trans-membrane delivery of a drug, the compound having a structure as set forth in Formula (VI): 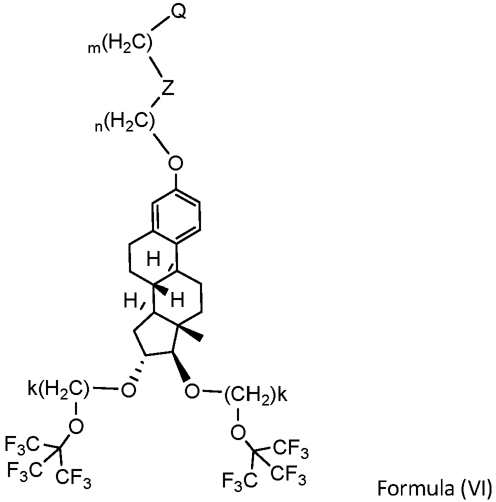 wherein
**n** and **m** are integers, each individually selected from null and 1, 2, 3, 4, 5, 6, 7, 8, 9, 10, 11, 12, 13, 14, 15, 16, 17, 18, 19 or 20;
**k** is an integer, selected from 2, 3, 4, 5, 6 or 7;
**Z** is null or -S-S-; and
**Q** is a linkage point to a drug;
or having a structure as set forth in Formula (VII): 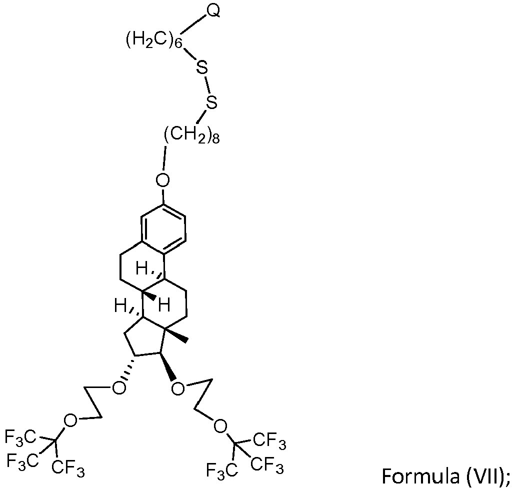
wherein Q is a linkage point to a drug;
or having a structure as set forth in Formula (VIIa): 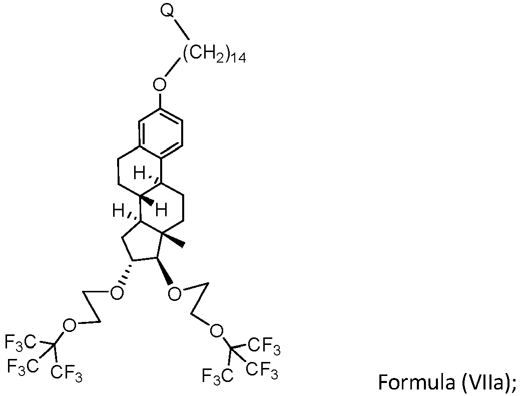
wherein Q is a linkage point to a drug;
or having a structure as set forth in Formula (VIII): 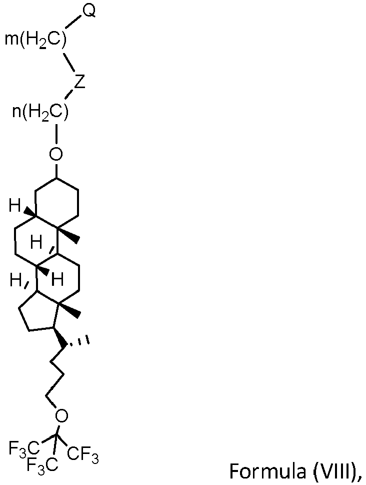 wherein
**n** and **m** are integers, each individually selected from null and 1, 2, 3, 4, 5, 6, 7, 8, 9, 10, 11, 12, 13, 14, 15, 16, 17, 18, 19 or 20;
**Z** is selected from null or -S-S-; and
**Q** is a linkage point to a drug;
or having a structure as set forth in Formula (IX): 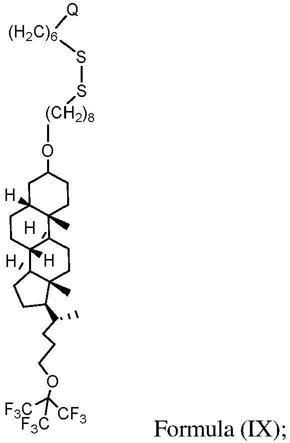
wherein Q is a linkage point to a drug;
or having a structure as set forth in Formula (IXa): 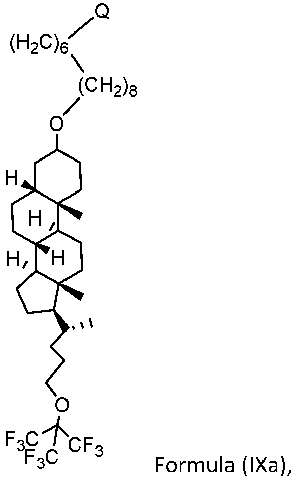
wherein Q is a linkage point to a drug;
or a pharmaceutically acceptable salt, hydrate, solvate or metal chelate thereof.

2. A pharmaceutical composition comprising a compound according to claim 1, and a pharmaceutically-acceptable salt or carrier.

3. A compound according to claim 1 for use in delivering a drug across a biological membrane.

4. . A compound for use according to claim 3, wherein the drug is a small-molecule drug or a macromolecule drug, selected from a natural or modified RNA or DNA, a small interfering RNA (siRNA), an antisense oligonucleotide (ASO) or a therapeutic protein.

5. A compound for use according to claim 4, wherein the therapeutic protein comprises a CRISPR protein, such as the Cas9 protein.

6. A compound for use according to claim 4, wherein the drug comprises a Dicer substrate, comprising double-stranded RNA of 25-30 nucleotides, that includes a sequence for silencing a target gene.

7. A compound for use according to any of claims 3 to 6, wherein said delivering of said drug is for treating a medical disorder, wherein the drug is useful for the treatment of the medical disorder.

8. A compound for use according to claim 7, wherein said medical disorder is cancer, a toxic insult, an ischemic disease, an infectious disease, a protein storage disease, trauma, an immune-mediated disease, a degenerative disease, or a neurodegenerative disorder such as Alzheimer's disease, motor neuron disease, Parkinson's disease, Huntington's disease, multiple sclerosis or Creutzfeldt-Jacob disease.

## Patentansprüche

1. Verbindung für eine transmembrane Bereitstellung eines Arzneimittels, wobei die Verbindung die in der Formel (VI) dargestellte Struktur hat: 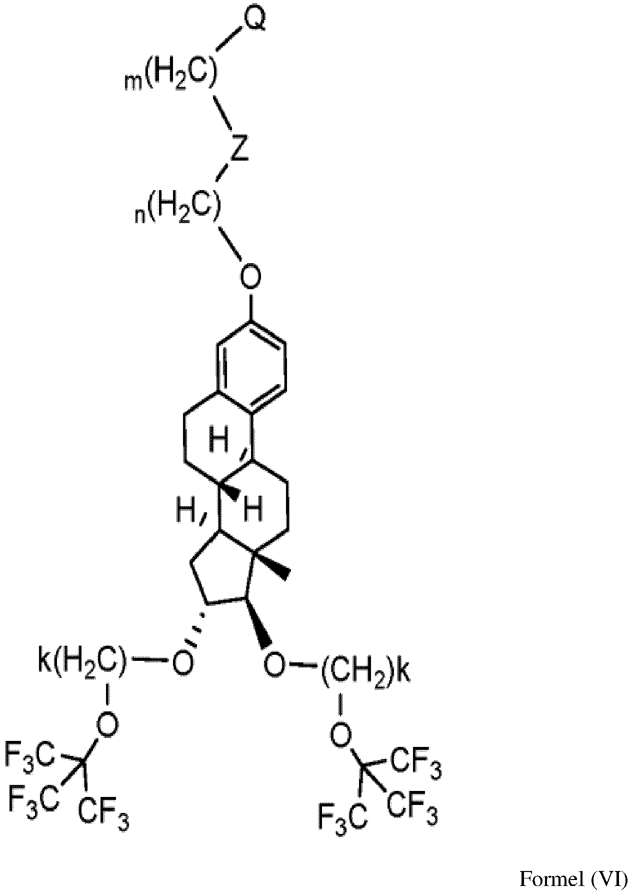 wobei
**n** und **m** ganze Zahlen sind, die jeweils einzeln aus null und 1, 2, 3, 4, 5, 6, 7, 8, 9, 10, 11, 12, 13, 14, 15, 16, 17, 18, 19 oder 20 ausgewählt sind
**k** eine ganze Zahl ist, die aus 2, 3, 4, 5, 6 oder 7 ausgewählt ist;
**Z** null oder -S-S- ist; und
**Q** ein Kopplungspunkt für ein Arzneimittel ist;
oder eine in der Formel (VII) dargestellte Struktur hat: 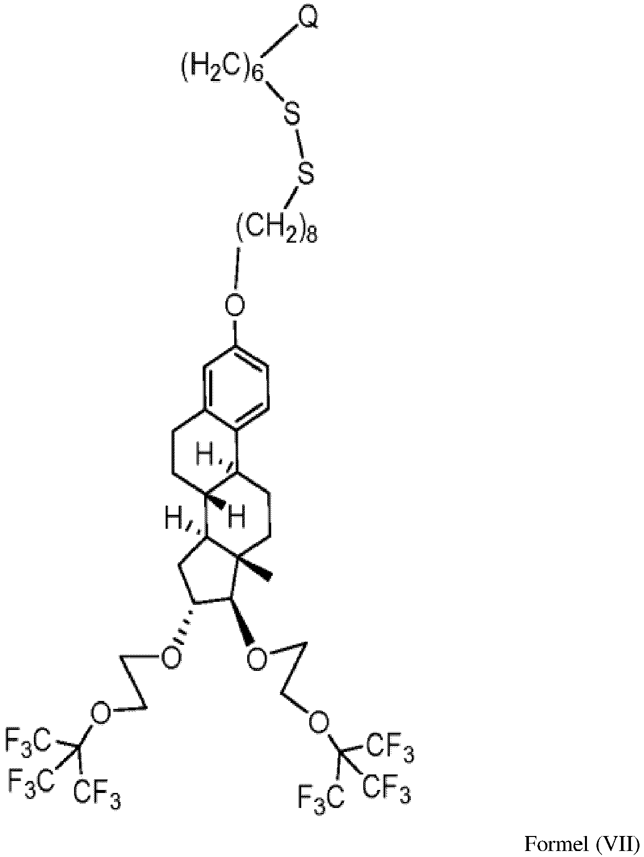
wobei **Q** ein Kopplungspunkt für ein Arzneimittel ist;
oder eine in der Formel (VIIa) dargestellte Struktur hat: 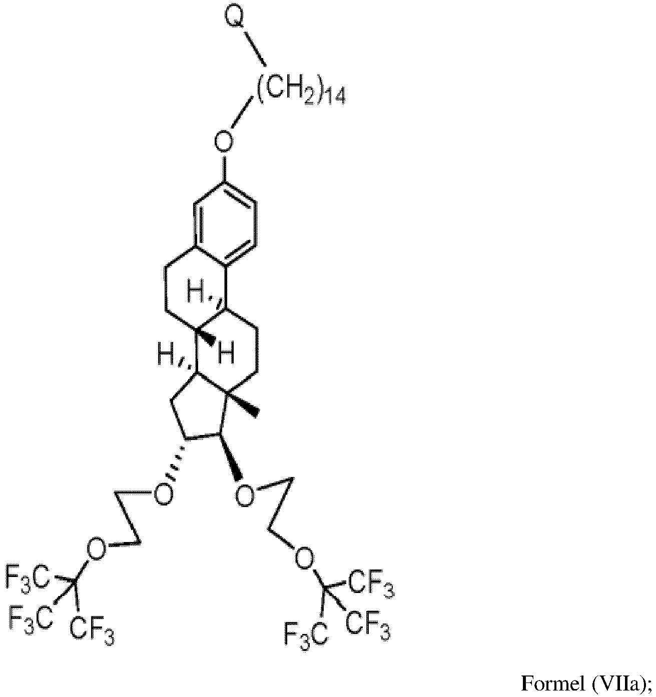
wobei **Q** ein Kopplungspunkt für ein Arzneimittel ist;
oder eine in der Formel (VIII) dargestellte Struktur hat: 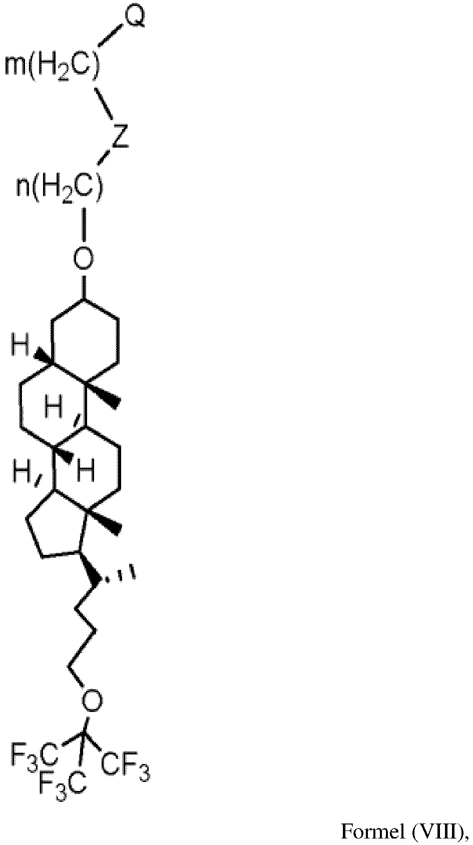 wobei
**n** und **m** ganze Zahlen sind, die jeweils einzeln aus null und 1, 2, 3, 4, 5, 6, 7, 8, 9, 10, 11, 12, 13, 14, 15, 16, 17, 18, 19 oder 20 ausgewählt sind
**Z** aus null oder -S-S- ausgewählt ist; und
**Q** ein Kopplungspunkt für ein Arzneimittel ist;
oder eine in der Formel (IX) dargestellte Struktur hat: 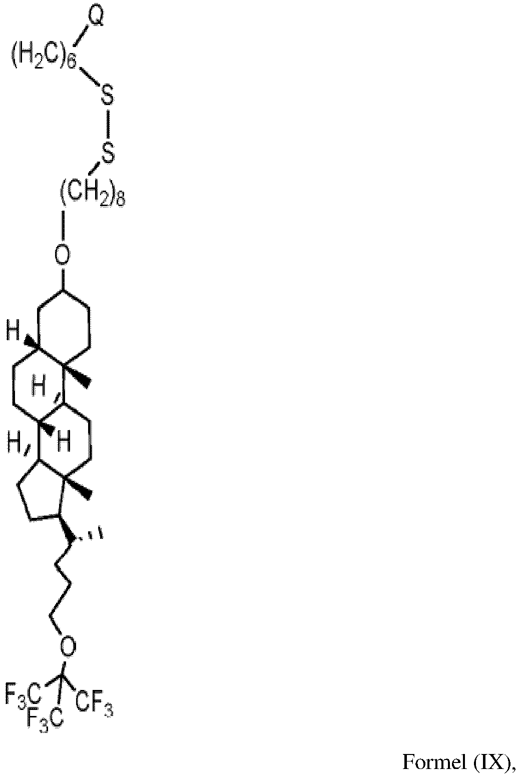
wobei **Q** ein Kopplungspunkt für ein Arzneimittel ist;
oder eine in der Formel (IXa) dargestellte Struktur hat: 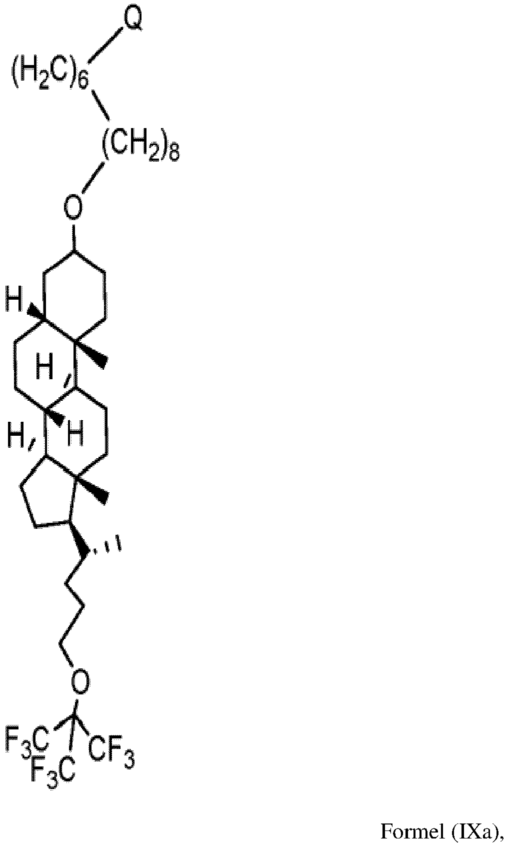
wobei **Q** ein Kopplungspunkt für ein Arzneimittel ist;
oder ein pharmazeutisch annehmbares Salz, Hydrat, Solvat oder Metall-Chelat davon.

2. Pharmazeutische Zusammensetzung, umfassend eine Verbindung nach Anspruch 1 und ein pharmazeutisch annehmbares Sals oder einen Träger davon.

3. Verbindung nach Anspruch 1 zur Anwendung beim Bereitstellen eines Arzneimittels durch eine biologische Membran hinweg.

4. Verbindung nach Anspruch 3, wobei das Arzneimittel ein Kleinmolekül-Arzneimittel oder ein Makromolekül-Arzneimittel ist, das aus einer natürlichen oder modifizierten RNA oder DNA, einer kleinen RNA-Interferenz (siRNA), einem Antisense-Oligonukleotid (ANO) oder einem therapeutischen Protein ausgewählt ist.

5. Verbindung nach Anspruch 4, wobei das therapeutische Protein ein CRISP-Protein, wie beispielsweise das Cas9-Protein, umfasst.

6. Verbindung nach Anspruch 4, wobei das Arzneimittel ein Dicer-Substrat umfasst, welches doppelständige RNA mit 25-30 Nukleotiden umfasst, welche eine Sequenz zum Ausschalten eines Zielgens aufweist.

7. Verbindung zur Anwendung nach einem der Ansprüche 3 bis 6, wobei ein Bereitstellen des Arzneimittels dem Behandeln einer medizinischen Störung dient, wobei das Arzneimittel zur Behandlung einer medizinischen Störung zweckdienlich ist.

8. Verbindung nach Anspruch 7, wobei die medizinische Störung eine Krebserkrankung, ein toxischer Insult, eine ischämische Erkrankung, eine Infektionskrankheit, eine Proteinspeicherkrankheit, ein Trauma, eine immunvermittelte Krankheit, eine degenerative Krankheit oder eine neurodegenerative Störung ist, wie beispielsweise Morbus Alzheimer, Motorneuronen-Krankheit, Morbus Parkinson, Morbus Huntington, Multiple Sklerose oder Creutzfeldt-Jakob-Krankheit.

## Revendications

1. Composé pour l'administration transmembranaire d'un médicament, le composé ayant une structure telle qu'indiquée dans la formule (VI) : 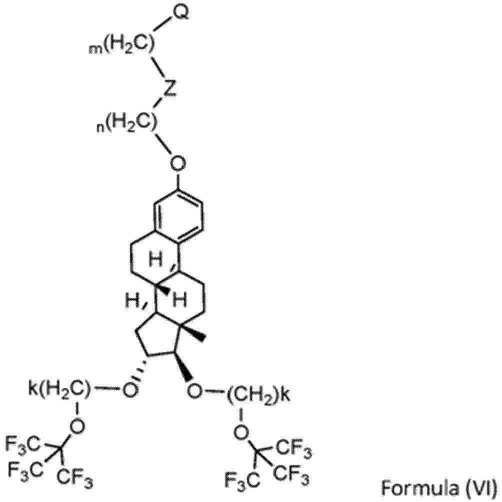 dans laquelle
**n** et **m** sont des nombres entiers, chacun individuellement choisi parmi zéro et 1, 2, 3, 4, 5, 6, 7, 8, 9, 10, 11, 12, 13, 14, 15, 16, 17, 18, 19 ou 20 ;
**k** est un nombre entier, choisi parmi 2, 3, 4, 5, 6 ou 7 ;
**Z** est nul ou -S-S- ; et
**Q** est un point de liaison à un médicament ;
ou ayant une structure telle qu'indiquée dans la formule (VII) : 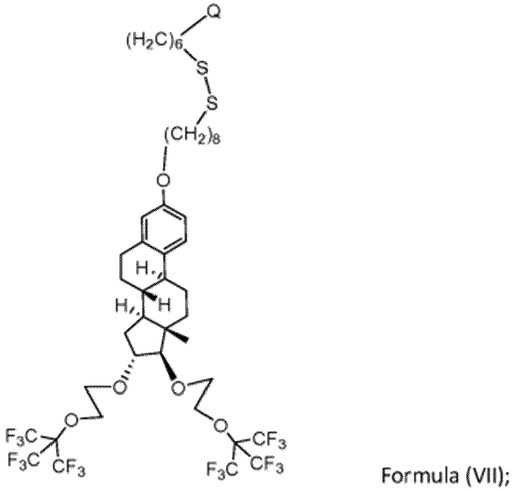
dans laquelle Q est un point de liaison à un médicament ;
ou ayant une structure telle qu'indiquée dans la Formule (VIIa) : 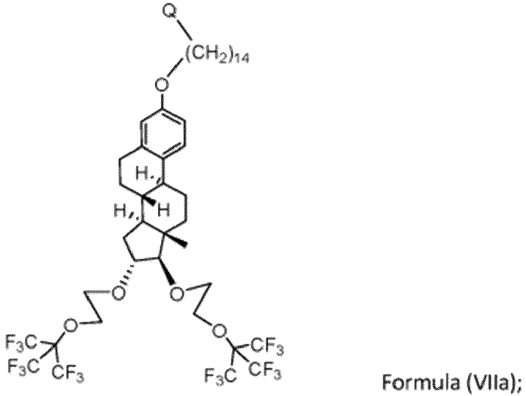
dans laquelle Q est un point de liaison à un médicament ;
ou ayant une structure telle qu'indiquée dans la formule (VIII) : 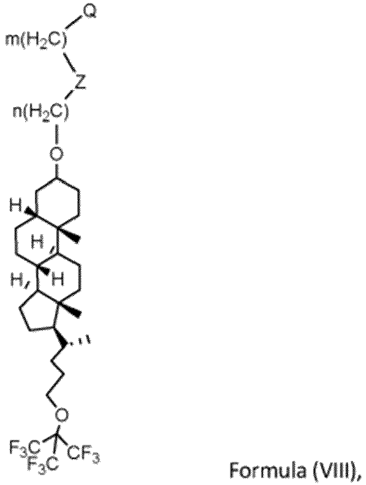 dans laquelle
**n** et **m** sont des nombres entiers, chacun individuellement choisi parmi zéro et 1, 2, 3, 4, 5, 6, 7, 8, 9, 10, 11, 12, 13, 14, 15, 16, 17, 18, 19 ou 20 ;
**Z** est choisiparmi zéro ou -S-S- ; et
**Q** est un point de liaison à un médicament ;
ou ayant une structure telle qu'indiquée dans la formule (IX) : 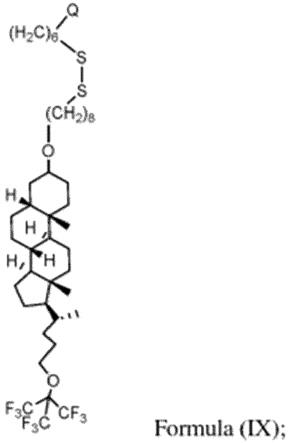
dans laquelle Q est un point de liaison à un médicament ;
ou ayant une structure telle qu'indiquée dans la formule (IXa) : 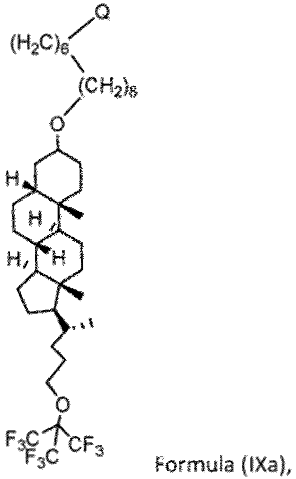
dans laquelle Q est un point de liaison à un médicament ;
ou un sel, hydrate, solvate ou chélate métallique pharmaceutiquement acceptable de celui-ci.

2. Composition pharmaceutique comprenant un composé selon la revendication 1, et un sel ou support pharmaceutiquement acceptable.

3. Composé selon la revendication 1 pour une utilisation pour délivrer un médicament à travers une membrane biologique.

4. Composé pour une utilisation selon la revendication 3, dans lequel le médicament est un médicament à petite molécule ou un médicament à macromolécule, choisi parmi un ARN ou ADN naturel ou modifié, un petit ARN interférent (ARNsi), un oligonucléotide antisens (ASO) ou une protéine thérapeutique.

5. Composé pour une utilisation selon la revendication 4, dans lequel la protéine thérapeutique comprend une protéine CRISPR, telle que la protéine Cas9.

6. Composé pour une utilisation selon la revendication 4, dans lequel le médicament comprend un substrat Dicer, comprenant un ARN double brin de 25 à 30 nucléotides, qui comprend une séquence pour faire taire un gène cible.

7. Composé pour une utilisation selon l'une quelconque des revendications 3 à 6, dans lequel ladite administration dudit médicament est destinée au traitement d'un trouble médical, le médicament étant utile pour le traitement du trouble médical.

8. Composé pour une utilisation selon la revendication 7, dans lequel ledit trouble médical est un cancer, une agression toxique, une maladie ischémique, une maladie infectieuse, une maladie de stockage de protéines, un traumatisme, une maladie à médiation immunitaire, une maladie dégénérative ou un trouble neurodégénératif comme la maladie d'Alzheimer, la maladie du motoneurone, la maladie de Parkinson, la maladie de Huntington, la sclérose en plaques ou la maladie de Creutzfeldt-Jacob.
